# EUROPEAN PATENT SPECIFICATION

(11) **EP 3 545 854 B1**
(45) Date of publication and mention of the grant of the patent: **30.11.2022**
(21) Application number: 17874853.9
(22) Date of filing: 21.11.2017
(51) Int. Cl.: A61B 10/00

(54) **PORTABLE BODY FLUID TESTING DEVICE**
TRAGBARE KÖRPERFLÜSSIGKEITSTESTVORRICHTUNG
DISPOSITIF DE TEST DE LIQUIDE CORPOREL PORTABLE

(30) Priority: 22.11.2016 KR 20160155781
(43) Date of publication of application: 02.10.2019
(73) Proprietor: Intin Co., LTD, Dong-gu Daegu 41111 (KR)
(72) Inventor: KIM, Ji Hoon, Daegu 42012 (KR)
(74) Representative: Kuhnen & Wacker Patent- und Rechtsanwaltsbüro PartG mbB
(86) International application number: PCT/KR2017/013224
(87) International publication number: WO 2018/097566

(56) References cited:
- WO-A2-2016/076669
- DE-U1-202016 100 042
- KR-A- 20130 130 443
- KR-A- 20160 098 215
- KR-B1- 101 533 107
- KR-U- 20000 003 733
- US-A1- 2014 120 982
- US-B1- 6 582 377

## Description

### Technical Field

The present invention relates generally to a portable body fluid testing device and, more particularly, to a portable body fluid testing device, which is easily attached to and detached from a portable terminal of a user, and is configured to be easily replaced, thereby being used hygienically.

### Background Art

Generally, people should visit a specialized medical institution to check their health state and body state. However, in order to receive diagnosis, people need to set aside time for visiting a specialized medical institution during busy work time, and go through complicated formalities and procedures. Particularly, even though most of these formalities are carried out, it is necessary to wait for a long time to receive the examination, while the actual examination and checkup are performed in a very short time. In other words, it takes a great deal of time and money to receive the examination and checkup, and many inconveniences follow. Thus, most people ignore endurable discomfort, and visit specialized medical institutions only when having unendurable pain or discomfort.

WO 2016076669 A2 discloses a method for monitoring ovulation using a smart phone, which can monitor saliva generated in a female ovulation period more precisely than a conventional ovulation predictor, using a smart phone, so as to more accurately predict whether ovulation occurs, and can provide monitored data to a user as various information through the smart phone using a smart phone application. The invention shown in WO 2016076669 comprises the steps of:
downloading and installing an ovulation app (application) on the smart phone (S100); executing the ovulation app (S110); analyzing a generated event by a control unit (180) (S130), and when an ovulation monitoring device is connected to the smart phone, driving a camera unit (140) by the control unit (180) so as to photograph saliva on a pallet member of the ovulation monitoring device where the saliva is placed; displaying the photographed image on a display unit (160) of the smart phone by the control unit (180) (S170); comparing the corresponding saliva image data with ovulation information data of other testees, included in the ovulation app, by the control unit (180) so as to analyze the data (S180); and outputting the analyzed data as an audio text or a video text indicating the infertile period, the transition period, or the ovulation period through the display unit (160) and a voice output unit (170) of the smart phone (S190).

In order to solve the inconvenience, techniques have been developed wherein various types of advanced equipment are used to frequently check one's body state and the result is transmitted to a specialized medical institution via a network. However, techniques that are commercialized and are actually used in practice are extremely rare.

Preferably, the use of a simple tester, which has been used for a long time, continues and is increasingly used by the younger generation due to ease of use, low time and cost, and fast results.

Such a tester detects hormones contained in secretions, or identifies changes in the state of the secretions by using secretions from a human body, e.g., saliva, urine, sweat, etc. The tester utilizes the fact that the body undergoes a change in the state of the component contained in the saliva when a certain condition, for example, a specific disease, an infection, an abnormality of the body such as an ovulatory period, or a specific event occurs. As a commonly used testing means, testers for pregnancy testing, diabetes diagnosis, and blood sugar checking are widely used in various forms. In particular, testers are often used by users because they are relatively accurate, easy to use, and affordable at times when the user wants to use it.

Recently, due to increase in the age at marriage, increase in the stress from social life, lack of exercise, etc., the number of couples who are infertile and have difficulty in becoming pregnant has increased, and thus the use of pregnancy tester has greatly increased. However, the conventional pregnancy tester determines only pregnancy or not and is not helpful for the user who is infertile and has difficulty in becoming pregnant. In other words, the conventional pregnancy tester determines only pregnancy or not, so important matters such as the woman's pregnancy probability, arrival of ovulation period, and the man's fertilization ability cannot be identified through the tester but can only identified only by a medical institution's consultation.

### Disclosure

### Technical Problem

Accordingly, an object of the present invention is intended to propose a portable body fluid testing device, the device serving as a tester which is portable and easy to use, whereby a user is able to quickly conduct self-testing and to identify the analysis result any time and any place by using body fluid of the user.

Further, another object of the present invention is intended to propose a portable body fluid testing device, in which both women and men can use the device for different test purposes, whereby a woman may identify the period of high pregnancy probability by checking her ovulation period and a man may identify his sperm viability, thereby increasing pregnancy probability.

Further, a further object of the present invention is intended to propose a portable body fluid testing device, which is easily attached to and detached from a portable terminal of a user, and is configured to be easily replaced, thereby being used hygienically.

### Technical Solution

In order to accomplish the above object, according to the present invention, there is provided a portable body fluid testing device as defined in claim 1.

The test unit may be configured such that the body is formed in a tubular shape having an inner space, and the inner space receives the magnifying lens group for magnifying the body fluid applied onto the sample part.

A side portion, as a circumferential surface, of the body may be formed of a non-permeable material that prevents transmission of light, or a non-permeable material may be applied to the side portion of the body to prevent transmission of light.

The contact portion and the flange are provided with a bonding member for fixing the test unit to the camera.

The testing device may further include a lower cover covering the contact portion or an upper cover covering the sample part.

The lower cover or the upper cover may be openably hinged to the body.

The body may be provided with a sheet guard formed to be higher than a surface of the sample part to surround a periphery of the sample part.

The user terminal may take at least one image of crystals formed upon drying of the body fluid, cells contained in the body fluid, and specific substances contained in the body fluid.

An object to be photographed by the user terminal may be any one of sperm, vaginal mucosa cells, oral cells, bacteria, and cells infected by the bacteria contained in the body fluid.

### Advantageous Effects

The portable body fluid testing device according to the present invention is advantageous in that by providing a tester which is portable and easy to use, a user is able to quickly conduct self-testing and to identify the analysis result any time and any place by using body fluid of the user.

The portable body fluid testing device according to the present invention is further advantageous in that both women and men can use the device for different test purposes, whereby a woman may identify the period of high pregnancy probability by checking her ovulation period and a man may identify his sperm viability, thereby increasing pregnancy probability.

The portable body fluid testing device according to the present invention is further advantageous in that it is easily attached to and detached from a portable terminal of a user, and is configured to be easily replaced, thereby being used hygienically.

### Description of Drawings

FIG. 1 is a view showing an example of use of a portable body fluid testing device.
FIG. 2 is an exemplary showing a cross-sectional shape of a test unit of the portable body fluid testing device according to the present invention.
FIG. 3 is an upper perspective view showing the test unit of the portable body fluid testing device.
FIG. 4 is a rear perspective view showing the test unit of the portable body fluid testing device.
FIG. 5 is a side view showing the test unit of the portable body fluid testing device.
FIG. 6 illustrates exemplary views showing change in body fluid for pattern analysis.
FIG. 7 illustrates exemplary views showing test images in which actual body fluid is used.
FIG. 8 is a view showing an example of a subdivision pattern for analysis of the test image.
FIG. 9 illustrates views showing an example of a case where the body fluid is semen or vaginal discharge.

### Best Mode

Hereinafter, exemplary embodiments of the present invention will be described with reference to the accompanying drawings. In the following description, the same elements will be designated by the same reference numerals although they are shown in different drawings. Further, in the following description of the present invention, a detailed description of known functions and configurations incorporated herein will be omitted when it may make the subject matter of the present invention rather unclear. In the drawing, certain features presented in the drawings are expanded, reduced, or simplified for easy explanation, and the drawings and the constituent elements may not be appropriately illustrated. However, those of ordinary skill in the art could easily understand such detailed matters.

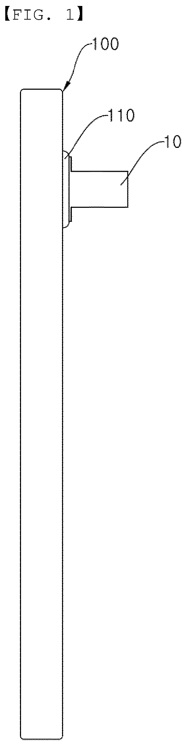

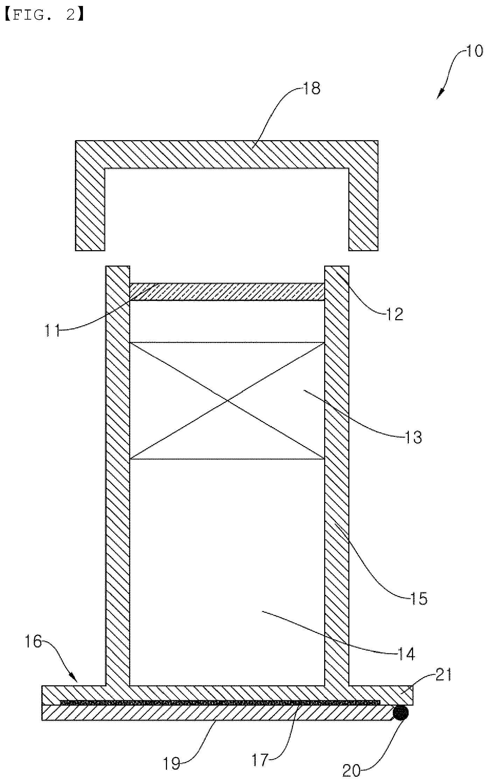

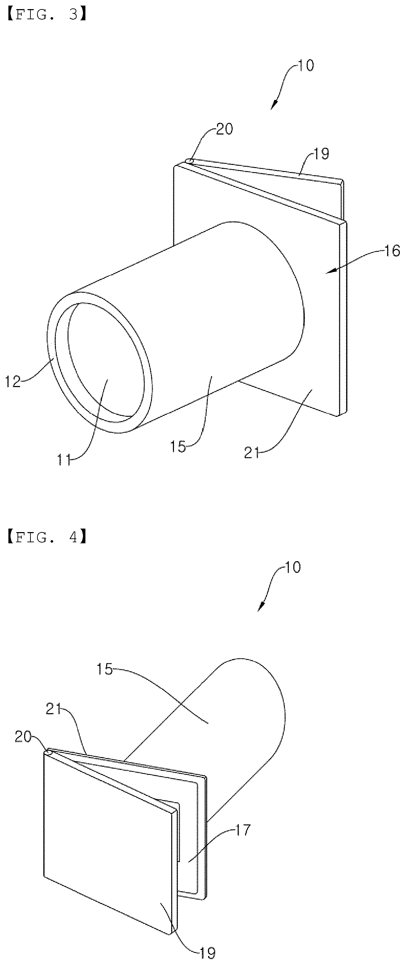

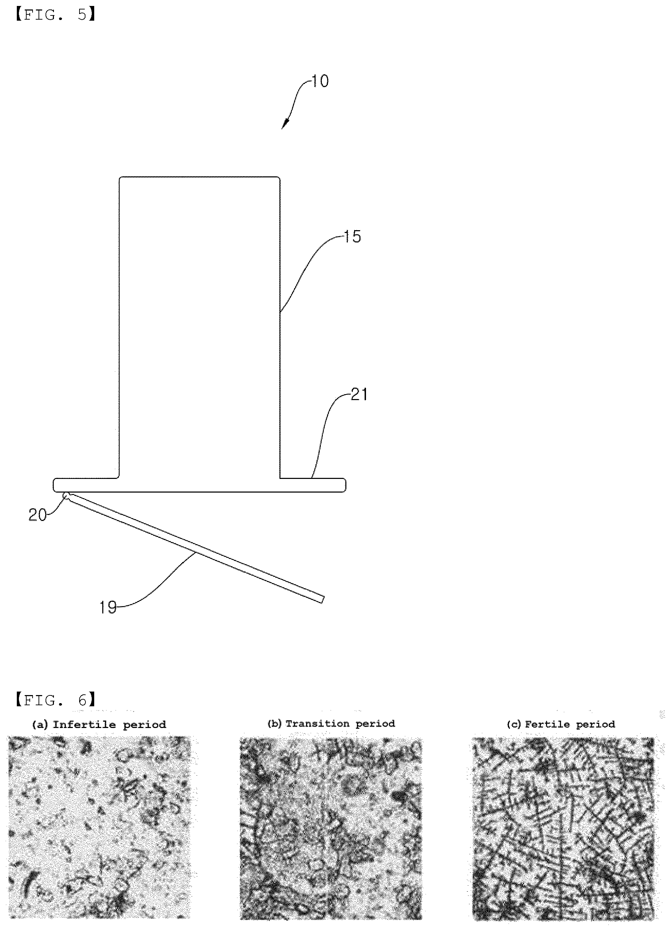

FIG. 1 is a view showing an example of use of a portable body fluid testing device, FIG. 2 is an exemplary showing a cross-sectional shape of a test unit of the portable body fluid testing device according to the present invention, FIG. 3 is an upper perspective view showing the test unit of the portable body fluid testing device, FIG. 4 is a rear perspective view showing the test unit of the portable body fluid testing device, and FIG. 5 is a side view showing the test unit of the portable body fluid testing device.

With reference to FIGS. 1 to 5, as shown in FIG. 1, a portable body fluid testing device of the present invention includes a test unit 10, and a user terminal 100.

The test unit 10 is attached to the user terminal 100, and serves as an objective for allowing the user terminal 100 to take an image of the body fluid used as a sample and analyze the same. To be more specific, the test unit 10 is attached to the user terminal 100, with the body fluid of a user applied onto the test unit. To achieve this, the test unit 10 includes a sample part 11 onto which body fluid is applied, and a magnifying lens group 13 for magnifying the by sample part 11 by combination of a camera 110 provided in the user terminal 100, and the detailed configuration thereof will be described in detail below. In particular, the test unit 10 is made of a lightweight, small-sized synthetic resin, glass, or a mixture of these materials and metal, and is manufactured inexpensively so as to be disposable after several uses. Accordingly, the user can replace the test unit 10 after one or several tests and use the test unit hygienically, and the test unit is made compact and lightweight, making it easy to carry and use. In particular, the test unit 10 is used by being attached to the user terminal 100 such as a smart terminal or a smart pad used by a user rather than a specialized examine device, thereby improving convenience.

The user terminal 100 is coupled to the test unit 10 onto which the body fluid is applied, and takes an image of the body fluid applied onto the test unit 10 by using a provided camera 110. Further, the captured image or image is analyzed by a pre-installed program (or an app), the user performs a desired test, and the analysis result is output or provided to a specialized medical institution. As the user terminal 100, any terminal with the camera 110, in which a program for image analysis can be installed, may be used. Typically, devices such as a smart phone, a smart pad, and a notebook are exemplified, but the present invention is not limited thereto. To achieve this, the test unit 10 is detachably coupled to the camera 110 of the user terminal 100 by a coupling member, and takes an image of a sample part 11 in a magnified manner by combination of a camera lens of the user terminal 100 and a magnifying lens group 13 of the test unit 10. The analysis based on the captured image will be described in more detail with reference to FIG. 6 and subsequent drawings.

The test unit 10 is as shown in FIGS. 2 to 5, and will be described in more detail with reference to these.

A body 15 of the test unit 10 is formed to be long in one direction, and is formed in an elliptical or polygonal tubular shape having a space therein. A first longitudinal end of the body 15 is provided with the sample part 11, and a second longitudinal end thereof facing the first longitudinal end is provided with the coupling member for coupling the test unit 10 to the user terminal 100. Also, the magnifying lens group 13 is accommodated in the space inside the body 15, and the remaining space serves as a barrel to maintain a predetermined distance between the camera 110 of the user terminal 100 and the magnifying lens group 13.

To be more specific, a first side of the body 15 is provided with the sample part 11 onto which body fluid is applied. Also, the sample part 11 provided in the body 15 also serves as a light inlet through which light is introduced so that the camera 110 can take an image of the body fluid applied onto the sample part 11. The sample part 11 is formed of a transparent synthetic resin or a glass material or an equivalent material thereof, and particularly, the sample part 11 itself may be one of the lenses included in the magnifying lens group 13. In other words, one side of the sample part 11 may be formed in a plane shape so as to allow the body fluid is uniformly applied, and the other side, that is, the direction facing the magnifying lens group 13, may be concave or convex so as to serve as a lens.

The body 15 corresponding to the edge portion of the sample part 11 is formed with a sheet guard 12 so as to contain body fluid applied onto the sample part 11 as shown in FIGS. 2 and 3 and to uniformly apply the body fluid. The sheet guard 12 may be integrally formed with either the body 15 or the sample part 11. Further, when the test unit 10 is provided with an upper cover 18, the sheet guard 12 maintains a gap between the sample part 11 and the upper cover 18 to prevent the inner surface of the upper cover 18 from contacting the sample part 11, and serves as a coupling portion for allowing the upper cover 18 to be coupled to the body. This is because the sheet guard 12 is formed extending from the sample part 11 or the body 15 in the longitudinal direction.

The magnifying lens group 13 includes a plurality of lenses, and may be composed of a plurality of lens combinations for magnification. In particular, as described above, the magnifying lens group 13 may include the sample part 11 as one of the plurality of lenses, but the present invention is not limited thereto. This magnifying lens group 13 compensates for an insufficient magnification ratio of the camera 110 provided in the user terminal 100 so that sufficient magnification can be achieved.

Meanwhile, the second longitudinal end of body 15, that is, the longitudinal end facing the sample part 11 is provided with a contact portion 16. The contact portion 16 is brought in contact with the camera 110 when the test unit 10 and the user terminal 100 are coupled to each other, and serves to fix the test unit 10 to the user terminal 100. To achieve this, the contact portion 16 is formed in a plane shape so as to be in close contact with the camera 110. In particular, the contact portion 16 may be provided on a surface thereof facing the user terminal 100 with a coupling means, for example, a bonding member 17. The bonding member 17 may be in the form that the adhesive tape is adhered to the surface of the contact portion 16 or the mucoadhesive is applied to the surface of the contact portion 16, but the present invention is not limited thereto. Other than the bonding member 17, any type of coupling member that can be attached to the camera 110 is available. In particular, the bonding member 17 is not provided on a portion of the surface of the contact portion 16 corresponding to the inner space of the body, but is provided on the remaining portion of the surface. Thus, when an image is taken by the camera 110, it is possible to prevent the image quality from being degraded by the bonding member 17. In addition, a portion of the contact portion 16 without having the bonding member 17 may be one of the plurality of lenses constituting the magnifying lens group 13, but the present invention is not limited thereto. Meanwhile, the contact portion 16 is formed with a flange 21 so as to be tightly and securely coupled with the camera 110 or the user terminal 100. The flange 21 is formed by extending from the body 15 in the radial direction, that is, in the direction perpendicular to the longitudinal direction, for widening the area of the contact portion 16.

The outer surface of the body 15 may be colored with an impermeable paint or the body 15 may be formed of an opaque material so that light can be transmitted only through a limited portion, for example, the sample portion 11. As a result, by allowing the light to be introduced only through the sample part 11, the sample can be clearly identified at the time of taking an image.

Meanwhile, the body 15 may be coupled with the upper cover 18 and a lower cover 19. The upper cover 18 can prevent foreign substances from being adsorbed or contaminated to the sample part 11, and the lower cover 19 can prevent deterioration of the adhesive force the bonding member 17 of the contact portion 16 due to contamination. The upper cover 18 and the lower cover 19 may be formed to be fitted to the longitudinal end of the body 15 formed in the shape of a cap (the same shape as the upper cover), or may be opened and closed in a state of being coupled to the body 15 like a hinge connection (connection shape of the lower cover) but the present invention is not limited thereto.

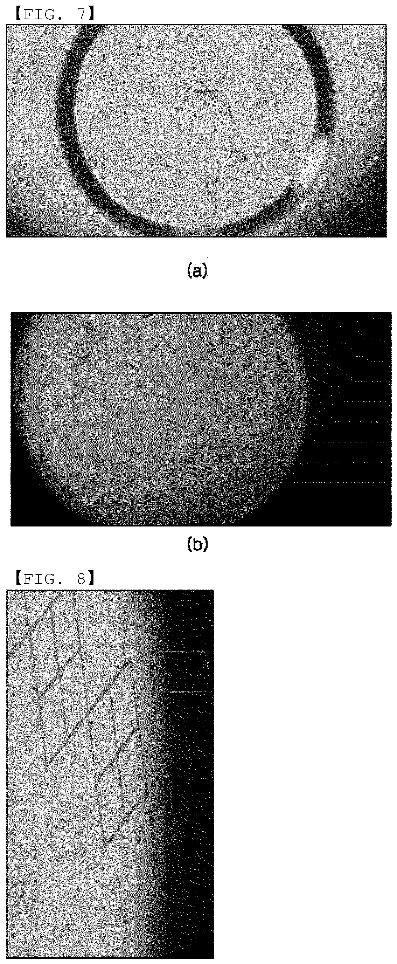

FIGS. 6 to 8 are exemplary views illustrating a pattern analysis method according to the present invention. FIG. 6 illustrates exemplary views showing change in body fluid for pattern analysis, and FIG. 7 illustrates exemplary views showing test images in which actual body fluid is used. Also, FIG. 8 is a view showing an example of a subdivision pattern for analysis of the test image.

With reference to FIGS. 6 to 8, the body fluid, particularly, the saliva may be used as an indicator of various conditions. The saliva of a woman in the fertile period shows a special sign distinctly. Specifically, due to the influence of estrogen which is a female hormone contained in the saliva of a woman, ovulation may be accurately predicted, and thus married couples suffering from infertility may increase pregnancy probability.

To be specific, when a woman is in the fertile period, as the ovulation date gets closer, the secretion rate of the estrogen increases, and also, the salt concentration of the saliva increases. Accordingly, when the saliva is dried for a particular time, different crystal structures of the dried saliva are shown depending on the infertile period, the transition period, and the fertile period.

For example, when drying and photographing the saliva of the woman in the infertile period during which ovulation does not occur, an image in which no distinct crystal structure is found is obtained as shown in FIG. 6 (a) .

FIG. 7(a) shows the actually obtained image. As shown in FIGS. 6(a) and 7(a), in the infertile period, no specific crystal structure is found, and only some salivary cells are identified.

In the transition period during which the infertile period transitions to the fertile period in which ovulation occurs, the saliva of the woman is slightly crystallized in the form as shown in FIG. 6(b). At this time, the form of the crystals is the form of salivary cells in circular and oblong shapes formed by electrolyte, mucus, enzyme, etc.

Last, in the fertile period during which ovulation occurs, the form in which the crystal extends in one direction, namely, a fern pattern clearly visible as shown in FIG. 6(c). FIG. 7(b) shows an image of actual saliva of the woman in the fertile period. As seen from this, the pattern distinctly distinguished from FIG. 7(a) is identified.

In the body fluid testing device of the present invention, the state of the saliva is analyzed to identify whether the user is in the fertile period, and the identified information is provided to the user as the analysis information.

In other words, the user terminal 100 performs image processing on the obtained test image to clarify the crystal line, and determines whether the crystal line is in the form of a closed curve line or in the form of the straight lines in the fern pattern as shown in FIG. 6(c). Particularly, the length and density of the linear pattern is identified. Consequently, the infertile period, the transition period, and the fertile period are determined and the result is provided to the user as the analysis information. Here, the image processing and the determining of the infertile period, the transition period, and the fertile period are possibly adjusted depending on the density and form of the pattern, and thus the detailed description thereof will be omitted.

Further, in image processing, the image is reprocessed in black and white, and the contrast of black and white is distinguished to determine the form of the line, whereby the length and the density is determined. Since various methods may be used, the detailed description thereof will be omitted.

In the meantime, in order to enhance accuracy of image processing and the determination, a subdivision pattern may be used as shown in FIG. 8. That is, how many straight lines or oval lines are present in the region (the region inside the closed line) defined by the pattern is identified, and the analysis information is generated.

In particular, the subdivision pattern may be provided in a printed form on the magnifying lens group 13 of the user terminal 100 or on the sample part 11, or a part of the analysis image may be partitioned in the subdivision pattern by the user terminal 100 for image processing, but the present invention is not limited thereto.

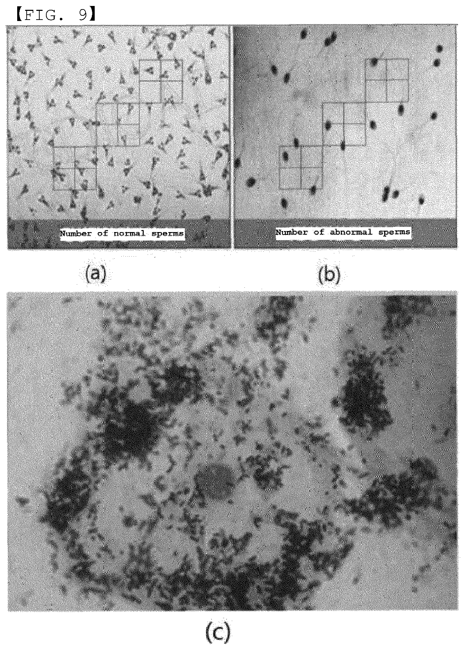

Meanwhile, FIG. 9 illustrates views showing an example of a case where the body fluid is semen or vaginal discharge.

The analysis information may be generated with respect to semen of a man. When using the body fluid testing device of the present invention with respect to semen of a man, matters that the number of sperms, sperm activity, and whether sperms have deformity may be identified. Particularly, after improving the state of sperms, it is possible to try to get pregnant.

In analyzing semen, the subdivision pattern may be used as shown in FIGS. 9(a) and (b). By determining the number, activity, and the form of sperms positioned within the grid pattern formed in the middle of FIGS. (a) and (b), the sperm characteristic is easily analyzed. To be more specific, all sperms distributed throughout the obtained image are not analyzed by the user terminal 100 or a medical institution system (not shown), sampling analysis may be performed on targets that are sperms in the region delimited by the subdivision pattern. According to the present invention, quick analysis is possible, and pattern processing load of the user terminal 100 may be reduced with more detailed analysis.

As an example of the subdivision pattern, although the grid form is shown in FIGS. 8 and 9, the pattern may be formed in various shapes, such as an oval, a circle, etc., and an arbitrary position may be specified as well as the center of the screen. Also, the image is checked, and the subdivision pattern is applied to the position with high density, whereby the analysis is conducted. The present invention is not limited thereto.

In order to analyze the sperm characteristic in the user terminal 100 of the present invention, a similar method to the above-described saliva analysis is performed. First, the user terminal 100 photographs the semen deposited on the sample part 11 to obtain the test image. The user terminal 100 computes the number of sperms, travel distances of particular sperms, the average of the travel distances, whether to rotate, the rotation speed, and the rotation radius identified within a particular region in the test image. Here, the user terminal 100 may obtain several test images for use, or may obtain the test image in the form of a video. Here, particular sperms in the image are randomly selected and the sperm characteristic of the randomly selected sperms is determined, whereby the state of sperm is identified. Also, the selection of multiple sperms for sampling may enhance accuracy of the analysis of the sperm characteristic.

Further, the user terminal 100 selects some sperms in the test image, the image of the selected sperms is processed in a clear pattern or in a black-and-white image, and then the result is compared with a pre-stored image or with a predetermined condition whether to have a shape corresponding thereto, whereby whether sperm is malformed is determined. For example, whether sperms in the test image have a normal form may be identified by determining the number of end portions (tail shape) per head (round part), whether the end portion is bent, and malformation of the head.

In particular, in this process, in order to facilitate selection and sampling of sperms, the subdivision pattern is applied as shown in FIG. 9, and the sperm characteristic may be analyzed by targeting the sperms positioned in the region defined by the subdivision pattern among sperms in the test image. Thus, selection of sperms for sampling may be easy, consistency of the analysis information may be maintained, and the load of the user terminal 100 due to the analysis may be reduced, whereby quick analysis is possible.

Also, as shown in FIG. 9(c), the vaginal discharge is observed to determine the presence of disease symptoms, such as bacterial vaginosis, vaginitis, and whether to perform diagnosis. Specifically, when the vaginal discharge is observed by using an examination device, only vaginal mucosa cells are observed for a normal case. However, as shown in FIG. 9(c), when a disease caused by bacterial infection occurs, substances such as those shown as black spots in FIG. 9(c) are identified. FIG. 9(c) shows the case in which secretion of anti-bacterial substances inside the vagina decreased resulting in an increase of vaginal acidity and overgrowth of anaerobic bacteria. The overgrown anaerobic bacteria is adsorbed on the surface of the vaginal mucosa cell and the vaginal mucosa cells are covered with black spots, and the symptom is identified by the examination device. As a result, according to the present invention, body secretion such as the saliva, the vaginal discharge, and the semen of man is photographed in a magnified manner, and an image analysis and a visual analysis are conducted such that various states, such as the presence of the disease, the name of the disease, the ovulation period, are possibly determined.

While the exemplary embodiments of the invention have been described above, the embodiments are only examples of the invention, and it will be understood by those skilled in the art that the invention can be modified in various forms. Therefore, modifications of the present invention are appreciated as included in the scope defined by the accompanying claims.

## Claims

1. A portable body fluid testing device comprising:
a test unit (10) configured such that a first side of a body formed to be long in one direction is provided with a sample part (11) onto which body fluid of a user is applied, a magnifying lens group (13) is provided, and a second side of the body is provided with a contact portion (16); and
a user terminal (100) having a camera (110) to be brought in contact with the contact portion (16) of the test unit (100) and configured to take an image of the body fluid applied onto the sample part (11), and configured for analyzing the image of the body fluid to determine a state of the body fluid,
**characterized by** the contact portion(16) having a flange (21) formed by extending from the body(15) in the radial direction and having a bonding member(17) for fixing the test unit(10) to the camera(110) formed on the surface of the contact portion(16) and the flange(21).

2. The testing device of claim 1, wherein the test unit (10) is configured such that the body is formed in a tubular shape having an inner space, and the inner space receives the magnifying lens group (13) for magnifying the body fluid applied onto the sample part (11).

3. The testing device of claim 2, wherein a side portion, as a circumferential surface, of the body is formed of a non-permeable material that prevents transmission of light, or a non-permeable material is applied to the side portion of the body to prevent transmission of light.

4. The testing device of claim 1, further comprising:
a lower cover (19) covering the contact portion (16) or an upper cover (18) covering the sample part (11).

5. The testing device of claim 4, wherein the lower cover (19) or the upper cover (18) is openably hinged to the body.

6. The testing device of claim 1, wherein the body is provided with a sheet guard (12) formed to be higher than a surface of the sample part (11) to surround a periphery of the sample part (11).

7. The testing device of claim 1, wherein the user terminal (100) is configured to take at least one image of crystals formed upon drying of the body fluid, cells contained in the body fluid, and specific substances contained in the body fluid.

8. The testing device of claim 7, wherein the user terminal (100) is configured to take an image of an object of any one of sperm, vaginal mucosa cells, oral cells, bacteria, and cells infected by the bacteria contained in the body fluid.

## Patentansprüche

1. Tragbare Körperflüssigkeitstestvorrichtung, aufweisend:
eine Testeinheit (10), die so konfiguriert ist, dass auf einer ersten Seite eines Gehäuses, das so geformt ist, dass es in einer Richtung lang ist, ein Probenteil (11), auf den Körperflüssigkeit eines Benutzers aufgebracht wird, vorhanden ist, und eine Vergrößerungslinsengruppe (13) vorhanden ist, und auf einer zweiten Seite des Gehäuses ein Kontaktabschnitt (16) vorhanden ist; und
ein Benutzerterminal (100) mit einer Kamera (110), die mit dem Kontaktabschnitt (16) der Testeinheit (100) in Kontakt gebracht wird und so konfiguriert ist, dass sie ein Bild der auf den Probenteil (11) aufgebrachten Körperflüssigkeit aufnimmt, und so konfiguriert ist, dass sie das Bild der Körperflüssigkeit analysiert, um einen Zustand der Körperflüssigkeit zu bestimmen,
**dadurch gekennzeichnet, dass** der Kontaktabschnitt (16) einen Flansch (21) aufweist, der durch eine Verlängerung von dem Gehäuse (15) weg in der radialen Richtung ausgebildet ist, und ein Verbindungselement (17) zum Befestigen der Testeinheit (10) an der Kamera (110), das an der Oberfläche des Kontaktabschnitts (16) und des Flansches (21) ausgebildet ist, aufweist.

2. Testvorrichtung nach Anspruch 1, wobei die Testeinheit (10) so konfiguriert ist, dass das Gehäuse in einer Röhrenform mit einem Innenraum ausgebildet ist, und wobei der Innenraum die Vergrößerungslinsengruppe (13) zum Vergrößern der auf den Probenteil (11) aufgebrachten Körperflüssigkeit aufnimmt.

3. Testvorrichtung nach Anspruch 2, wobei ein Seitenabschnitt, als eine Umfangsfläche, des Gehäuses aus einem undurchlässigen Material ausgebildet ist, das eine Lichttransmission verhindert, oder ein undurchlässiges Material auf den Seitenabschnitt des Gehäuses aufgebracht ist, um eine Lichttransmission zu verhindern.

4. Testvorrichtung nach Anspruch 1, ferner aufweisend:
eine untere Abdeckung (19), die den Kontaktabschnitt (16) abdeckt, oder eine obere Abdeckung (18), die den Probenteil (11) abdeckt.

5. Testvorrichtung nach Anspruch 4, wobei die untere Abdeckung (19) oder die obere Abdeckung (18) aufklappbar und mit einem Scharnier an dem Gehäuse befestigt ist.

6. Testvorrichtung nach Anspruch 1, wobei das Gehäuse ein Schutzblech (12) aufweist, das so gebildet ist, dass es höher als eine Oberfläche des Probenteils (11) ist, um einen Randbereich des Probenteils (11) zu umgeben.

7. Testvorrichtung nach Anspruch 1, wobei das Benutzerterminal (100) so konfiguriert ist, dass es zumindest ein Bild von Kristallen, die beim Trocknen der Körperflüssigkeit gebildet werden, Zellen, die in der Körperflüssigkeit enthalten sind, und bestimmten Substanzen, die in der Körperflüssigkeit enthalten sind, aufnimmt.

8. Testvorrichtung nach Anspruch 7, wobei das Benutzerterminal (100) konfiguriert ist zum Aufnehmen eines Bilds eines Objekts aus Sperma oder Vaginalschleimhautzellen oder Oralzellen oder Bakterien oder Zellen, die von in der Körperflüssigkeit enthaltenen Bakterien, infiziert sind.

## Revendications

1. Dispositif portable de test de fluide corporel comprenant :
- une unité de test (10) configurée de façon qu'un premier côté du corps de forme allongée dans une direction soit muni d'une partie d'échantillons (11) recevant le fluide corporel d'un utilisateur,
- un groupe de lentilles d'agrandissement (13), et
- un second côté du corps est muni d'une partie de contact (16), et
- un terminal d'utilisateur (100) ayant une caméra (110) pour être mis en contact avec la partie de contact (16) de l'unité de test (100) et configurée pour prendre une image du fluide corporel appliqué sur la partie échantillon (11) et configurée pour analyser l'image du fluide corporel pour déterminer l'état du fluide corporel,
dispositif **caractérisé en ce que**
la partie de contact (16) a une bride (21) formée par l'extension du corps (15) dans la direction radiale et un élément de liaison (17) pour fixer l'unité de test (10) sur la caméra (110), formé sur la surface de la partie de contact (16) et de la bride (21).

2. Dispositif de test selon la revendication 1,
dans lequel
l'unité de test (10) est configurée de façon que le corps soit de forme tubulaire ayant un espace intérieur et cet espace intérieur reçoit le groupe de lentilles de grossissement (13) pour grossir le fluide corporel appliqué sur la partie échantillon (11).

3. Dispositif de test selon la revendication 2,
dans lequel
une partie de côté comme surface périphérique du corps est formée en une matière non transparente évitant la transmission de la lumière ou en une matière non transparente appliquée sur la partie de côté du corps pour éviter la transmission de la lumière.

4. Dispositif de test selon la revendication 1,
comprenant en outre
- un couvercle inférieur (19) couvrant la partie de contact (16) ou un couvercle supérieur (18) couvrant la partie échantillon (11).

5. Dispositif de test selon la revendication 4,
dans lequel
le couvercle inférieur (19) ou le couvercle supérieur (18) est relié par une articulation au corps de façon à pouvoir s'ouvrir.

6. Dispositif de test selon la revendication 1,
dans lequel
le corps comporte une feuille de garde (12) formée pour dépasser en hauteur la surface de la partie échantillon (11) pour entourer la périphérie de la partie échantillon (11).

7. Dispositif de test selon la revendication 1,
dans lequel
le terminal d'utilisateur (100) est configuré pour prendre au moins une image des cristaux formés par le séchage du fluide corporel, des cellules contenues dans le fluide corporel et des substances spécifiques contenues dans le fluide corporel.

8. Dispositif de test selon la revendication 7,
dans lequel
le terminal d'utilisateur (100) est configuré pour prendre une image d'un objet de n'importe quel élément suivant : sperme, cellule mucosi-vaginale, cellule buccale, bactérie et cellule infectée par les bactéries contenues dans le fluide corporel.
